# DEMANDE DE BREVET EUROPEEN

(11) **EP 4 151 161 A1**
(43) Date de publication de la demande: **22.03.2023**
(21) Numéro de dépôt: 21198058.6
(22) Date de dépôt: 21.09.2021
(51) Int. Cl.: A61B 17/11

(54) **DISPOSITIF ANCILLAIRE ET KIT POUR ANASTOMOSE**

(71) Demandeur: Easy Vascular Cure, 94000 Créteil (FR)
(72) Inventeur: ABOU TAAM, Salam, 94000 Créteil (FR)
(74) Mandataire: Ipside

(57) **Abrégé**

La présente invention a comme objet de proposer un dispositif ancillaire apte à assister les deux procédures d'anastomose nécessaires pour remplacer un segment de conduit corporel par une prothèse, et la liaison de ladite prothèse sans besoin de suture. L'invention concerne aussi un kit optimisé pour anastomose comportant ledit dispositif ancillaire.

En particulier, l'invention propose un dispositif (100) ancillaire pour anastomose, ledit dispositif (100) comportant un corps allongé pourvu d'un canal (12) longitudinal et traversant destiné à recevoir à son intérieur une prothèse (3) tubulaire, le corps du dispositif comportant d'un côté proximal une partie de manipulation (10) et d'un côté distal un ensemble de conformation (20) formé par une pluralité des lamelles (22) définissant une zone de compression (21) de ladite prothèse, caractérise en ce que :
-le corps du dispositif comporte une ouverture longitudinale refermable donnant accès au canal (12) longitudinal destiné à recevoir ladite prothèse (3).

## Description

### Domaine de l'invention

La présente invention concerne le domaine de dispositifs d'aide d'intervention en anastomose. Plus particulièrement, l'invention concerne un dispositif ancillaire de pose d'un ensemble de liaison dans un conduit corporel pour la réalisation d'une suture entre deux conduits extracorporels et corporels. L'invention concerne également un kit comportant ledit dispositif ancillaire et au moins un ensemble de liaison comportant un manchon d'élargissement radial à picots et un ballon d'inflation.

### Etat de la technique

Une anastomose désigne dans la présente invention une connexion entre deux structures corporelles tubulaires, tel que des vaisseaux sanguins, des bronches ou des segments d'intestin. Les anastomoses sont très communes pour remédier à des problèmes vasculaires, par exemple pour traiter une artère thrombosée ou anévrismale en sectionnant et retirant sa partie pathologique, et reconnectant ensuite les deux segments de l'artère au moyen d'une prothèse.

Pour reconnecter les segments de vaisseaux, il est connu de réaliser une suture manuelle chirurgicale. Ce type d'anastomose qui est le plus fréquent en chirurgie vasculaire, permet de répondre à la majorité des situations cliniques de réparation artérielle. Cependant, on observe que le temps opératoire nécessaire à la réalisation d'une telle suture manuelle est relativement long. Or, lors d'une réparation vasculaire par exemple, l'anastomose nécessite un clampage vasculaire pour éviter toute hémorragie. La durée de ce clampage d'une part, constitue une épreuve d'effort cardiaque pour le patient pouvant conduire à un syndrome coronarien et d'autre part, génère une ischémie des tissus vascularisés par l'axe artériel d'aval.

En outre, une telle intervention manuelle impose une large accessibilité afin que le praticien puisse réaliser les gestes opératoires nécessaires à la liaison des deux segments entre eux. Cette accessibilité est aussi déterminante que la dextérité du praticien dans le bon accomplissement de la suture.

Toutefois, une telle accessibilité ne peut être obtenue qu'au travers d'une surface opératoire exposée importante, laquelle entraîne par exemple dans le cadre de la chirurgie aortique, des répercussions négatives sur les douleurs post-opératoires, la reprise du transit intestinal, les pneumopathies, les coronaropathies et les durées de séjour hospitalier. La chirurgie conventionnelle par mini-abord, ainsi que la chirurgie aortique laparoscopique, permettent de résoudre de tels inconvénients et de diminuer les répercussions suscitées pour le patient.

Néanmoins, la chirurgie conventionnelle par mini-abord et la chirurgie aortique laparoscopique offrent des conditions d'accessibilité réduites, rendant difficile la réalisation des gestes chirurgicaux nécessaires à la suture vasculaire en retirant une section du vaisseau et connectant les deux extrémités produites à l'aide d'une prothèse.

Afin de substituer cette suture manuelle, il est connu d'utiliser un stent (ou connecteur) très particulier en termes de design et de fonction se présentant sous la forme d'un manchon métallique expansible présentant des picots au niveau de sa surface extérieure. Ce stent à picots est monté sur un ballon d'inflation et introduit à une extrémité de la prothèse à connecter avec le conduit corporel. Lorsque l'extrémité de la prothèse est insérée dans le conduit, le ballon est gonflé de sorte à augmenter le diamètre du manchon, les picots perforent ainsi la prothèse et le conduit corporel pour assurer la liaison entre ces deux éléments.

Cette technique de liaison est très avantageuse mais pose quelques problèmes dans la pratique, notamment pour positionner le stent à picots dans la prothèse et le conduit corporel. Le brevet EP 2638 869 B1 propose un dispositif ancillaire spécialement conçu pour faciliter une anastomose à l'aide d'un stent à picots et d'un ballon d'inflation. En particulier, ce document propose un dispositif ancillaire comportant un corps tubulaire creux comportant une partie de manipulation et un ensemble de conformation, ledit corps comportant un orifice traversant dans lequel est insérée ladite prothèse avec le stent à picots monté sur le ballon. L'ensemble de conformation permet de compresser la prothèse suffisamment pour permettre son insertion dans le conduit corporel, tout en limitant ladite compression pour ne pas endommager les picots ou la prothèse avant l'étape de gonflage. Ce document suggère également d'utiliser une enveloppe de protection entourant le stent à picots. Le stent et la prothèse sont ainsi protégés lors de sa manipulation jusqu'à son insertion dans le conduit corporel. Bien entendu, l'enveloppe doit être retirée avant l'étape de gonflage.

En pratique, ledit dispositif ancillaire est très avantageux pour assister à la pose de la prothèse et du stent à picots lors de la première anastomose. Toutefois, ce dispositif est inadapté à assister la deuxième anastomose. C'est-à-dire, lorsqu'un segment d'un conduit corporel est remplacé par une prothèse, la procédure de remplacement complète requiert de deux connexions, une première anastomose pour relier une extrémité de la prothèse à l'extrémité dite « haute » du conduit corporel, et une deuxième anastomose pour relier l'extrémité dite « basse » du conduit corporel et l'autre extrémité de la prothèse. L'inconvenant dudit dispositif ancillaire de l'état de la technique est donc de ne pas être apte à assister dans la deuxième anastomose.

En effet, un segment à remplacer du conduit corporel, et donc de la prothèse, présente souvent une longueur limitée parfois de l'ordre de deux centimètres. Une fois que la première anastomose est réalisée, la longueur du segment libre de la prothèse est très limitée. D'autre part, le corps du dispositif ancillaire à une longueur d'environ 20 cm, et la prothèse est introduite dans ledit dispositif par une entrée opposée à la zone de compression de la prothèse et à partir de laquelle ladite prothèse est introduite dans le conduit. Il n'est donc pas possible d'introduire la deuxième extrémité de la prothèse tout le long du dispositif ancillaire, et jusqu'à ladite zone de compression. De plus, certaines difficultés sont observées pour bien positionner le ballon avec le stent dans le dispositif ancillaire, et pour retirer l'enveloppe protectrice du ballon.

### Exposé de l'invention

La présente invention a comme objet de proposer un dispositif ancillaire apte à assister les deux procédures d'anastomose nécessaires pour remplacer un segment de conduit corporel par une prothèse, et la liaison de ladite prothèse sans besoin de suture. L'invention concerne aussi un kit optimisé pour anastomose comportant ledit dispositif ancillaire.

À cette fin l'invention propose un dispositif ancillaire pour anastomose, ledit dispositif comportant un corps allongé pourvu d'un canal longitudinal et traversant destiné à recevoir à son intérieur une prothèse tubulaire, le corps du dispositif comportant d'un côté proximal une partie de manipulation et d'un côté distal un ensemble de conformation formé par une pluralité des lamelles définissant une zone de compression de ladite prothèse, caractérise en ce que :
- le corps du dispositif comporte une ouverture longitudinale refermable donnant accès au canal longitudinal destiné à recevoir ladite prothèse.

De manière avantageuse, l'ouverture longitudinale permet de donner accès au canal du dispositif, et donc de positionner précisément la prothèse au niveau de l'ensemble de conformation, notamment lorsque la longueur de la prothèse est insuffisante pour manœuvrer ladite prothèse après la première anastomose. De plus, cette ouverture refermable permet de désengager facilement le dispositif ancillaire suite à l'insertion de la deuxième extrémité de la prothèse dans le conduit corporel, lors de la deuxième anastomose.

Dans un mode de réalisation non limitatif, le dispositif ancillaire peut également comporter les caractéristiques techniques suivantes dans toute combinaison techniquement opérable :
- l'ouverture longitudinale est configurée comme une ouverture articulée ouvrant en deux parties ledit corps, et de préférence en deux parties symétriques ;
- le dispositif comporte une configuration ouverte, et une configuration fermée définies respectivement par l'ouverture ou la fermeture de ladite ouverture longitudinale, et un moyen de verrouillage de ladite configuration fermée ;
- les lamelles de l'ensemble de conformation sont disposées de manière circonférentielle et selon des intervalles prédéfinis autour de la partie de manipulation et suivant un axe longitudinal du dispositif, et dans lequel dispositif les lamelles comportent une position relâchée et une position serrée définissant respectivement un diamètre initial et un diamètre réduit du canal recevant la prothèse au niveau de ladite zone de compression ;

- la partie de manipulation comporte une ou plusieurs parties en saillie au niveau d'une zone de jonction avec les lamelles de l'ensemble de conformation, ladite partie en saillie permettant de comprimer radialement les lamelles lorsqu'elle est poussée vers l'intérieur du dispositif ;
- le dispositif comporte une bague extérieure mobile en translation le long du corps du dispositif, ladite bague étant apte à s'engager sur la ou les parties en saillie pour activer la position fermée de lamelles. Cette bague extérieure mobile permet aussi de solidariser en position fermée les deux parties symétriques du corps ;
- ledit dispositif comporte un moyen d'actionnement configuré pour comprimer radialement lesdites lamelles et activer leur position serrée, tel que ladite bague extérieure ;

L'invention concerne également un kit pour anastomose optimisé pour coopérer avec le dispositif ancillaire de l'invention, ledit kit comportant :
- un dispositif ancillaire selon l'une des revendications précédentes ;
- un ensemble de liaison comportant au moins un manchon d'élargissement radial à picots et un ballon d'inflation ; et de préférence
- une prothèse ou substitut vasculaire.

Dans un mode de réalisation, le kit comporte un système de guidage d'insertion comportant un jeu des éléments d'arrêt sur le corps du dispositif ancillaire et sur l'ensemble de liaison, configurés et disposés pour limiter la profondeur d'insertion de l'ensemble de liaison dans le canal du dispositif ancillaire.

Dans un mode de réalisation, le ballon d'inflation comporte un cathéter pour relier ledit ballon à une seringue d'inflation, et ledit cathéter comporte une ou des ailettes configurées pour s'ancrer au corps du dispositif ancillaire ou à la prothèse, et limiter une profondeur d'insertion dudit ballon d'inflation dans le canal du dispositif ancillaire. De préférence, les ailettes comportent des crochets périphériques configurés pour s'accrocher à une extrémité proximale de la partie de manipulation du dispositif ancillaire. De préférence, les ailettes sont mobiles en translation le long du cathéter et comportent une configuration verrouillée et une configuration déverrouillée, limitant ou permettant respectivement une mobilité des ailettes.

Dans un mode de réalisation, le kit comporte une enveloppe de protection pelable ou sécable entourant le manchon d'élargissement radial à picots. À cette fin, l'enveloppe comporte au moins un axe longitudinal pelable ou sécable.

Enfin, le kit comporte également un testeur de diamètre comportant au moins un corps tubulaire de diamètre prédéfini relié à une poignée, et de préférence une pluralité des corps tubulaires reliés respectivement à une poignée. Lesdits corps tubulaires étant destinés à être introduits dans un conduit corporel pour déterminer un diamètre de prothèse à insérer, ledit diamètre étant identifié lorsque l'un desdits corps tubulaires est introduit dans le conduit corporel avec un jeu limité.

### Brève description des dessins

L'invention sera mieux comprise à la lecture de la description qui suit et à l'examen des figures qui l'accompagnent. Celles-ci ne sont présentées qu'à titre indicatif et nullement limitatif de l'invention.
Fig. 1 Un exemple de kit d'anastomose comportant un dispositif ancillaire selon l'invention
Fig. 2 Une représentation schématique de la position relâchée (détail a) et de la position serrée (détail b) de l'ensemble de conformation du dispositif ancillaire de l'invention.
Fig. 3 Une représentation schématique du retrait du dispositif ancillaire après la première anastomose.
Fig.4 Une représentation schématique du dispositif ancillaire de l'invention en configuration ouverte et de la réception de la prothèse après la première anastomose.
Fig.5 Une représentation schématique du dispositif ancillaire de l'invention en configuration ouverte et d'une variante de réception de la prothèse après la première anastomose.
Fig.6 Une représentation schématique de l'élargissement radial d'un stent à picots dans un conduit corporel.
Fig. 7 Une représentation schématique du ballon d'inflation pourvu d'un élément d'arrêt du kit d'anastomose selon l'invention.
Fig. 8 Une représentation schématique du kit de l'invention montrant l'enveloppe de protection insérée dans le canal du dispositif ancillaire

La présente invention concerne un dispositif d'aide à la pose d'une prothèse dans un conduit corporel, ledit dispositif étant référé comme dispositif ancillaire dans la présente invention. En particulier, l'invention a comme but de proposer un dispositif ancillaire configuré pour assister dans la pose de deux extrémités opposées d'une prothèse et leur jonction avec un conduit corporel. L'invention concerne également un kit d'anastomose sans suture comportant ledit dispositif ancillaire.

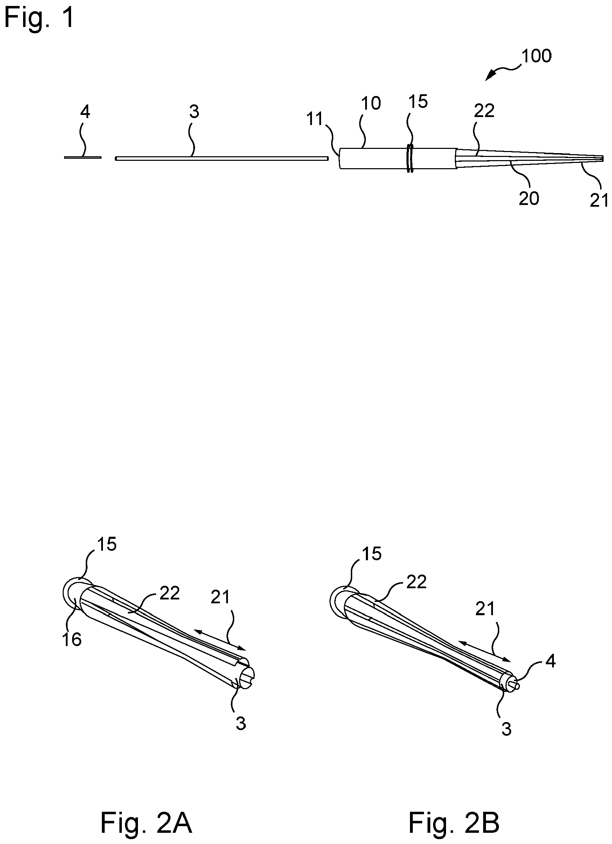

La figure 1 illustre de gauche à droite, un ensemble de liaison 4 (détail non visible), une prothèse 3, et le dispositif ancillaire 100 de l'invention dans une configuration fermée.

Le dispositif ancillaire 100 est un outil permettant la manipulation de la prothèse 3 en toute sécurité, ainsi que facilitant sa connexion au conduit corporel au moyen de l'ensemble de liaison 4.

Le dispositif 100 ancillaire comporte un corps allongé comportant d'un côté proximal une partie de manipulation 10, et d'un autre côté distal un ensemble de conformation 20 pourvu d'une zone de compression 21 à une extrémité distale du dispositif. Le corps du dispositif comporte également un canal longitudinal, destiné à recevoir la prothèse 3, et traversant ladite partie de manipulation et l'ensemble de conformation. Dans le mode de réalisation illustré, la partie de manipulation 10 se présente comme une enveloppe tubulaire creuse, l'intérieur creux de son corps définissant le canal au niveau de ladite partie de manipulation. La prothèse est notamment insérée dans le canal du dispositif à partir d'une entrée 11 latérale de la partie de manipulation 10 et jusqu'à positionner une extrémité de la prothèse 3 au niveau de ladite zone de compression 21, cette dernière comportant un diamètre de canal linéaire de diamètre dégressif selon l'actionnement de l'ensemble de conformation pour compresser ladite prothèse jusqu'à l'obtention d'un diamètre réduit prédéfini. Alternativement, la prothèse peut être introduite à partir de la zone de compression tel qu'il sera détaillé plus loin dans le texte.

En particulier, l'ensemble de conformation 20 permet de pincer une extrémité de la prothèse 3 pour la comprimer radialement et faciliter son insertion dans le conduit 30 corporel. À cette fin, l'ensemble de conformation comporte une pluralité des lamelles 22 disposées de manière circonférentielle autour de la partie de manipulation 10 et suivant un axe longitudinal du dispositif. Lesdites lamelles 22 prolongent le corps tubulaire de la partie de manipulation 10 et permettent de définir le canal traversant au niveau de l'ensemble de conformation 20. Tel qu'illustré sur la figure 2, les lamelles sont disposées à des intervalles réguliers, en laissant des espaces vides entre les lamelles.

L'ensemble de conformation 20 comporte une position relâchée (Figure 2, détail a) et une position serrée (Figure 2, détail b). Dans la position relâchée, les lamelles 22 définissent le diamètre du canal 12 permettant d'insérer facilement la prothèse jusqu'à la zone de compression 21. Dans la position serrée, les lamelles 22 sont resserrées, et le diamètre du canal est réduit, notamment au niveau de la zone de compression, de sorte que les parois intérieures de lamelles compriment la prothèse. De préférence, le diamètre du canal transversant est constant à la zone de compression 21 et de sorte à permettre une compression uniforme de la prothèse dans cette zone. Le diamètre du canal 12 traversant dans le reste du dispositif est supérieur à un diamètre de la prothèse, réalisant ainsi une butée empêchant de trop introduire le dispositif dans le conduit corporel pour que l'ensemble soit introduit sans endommager ou compresser ledit conduit corporel. Les dimensions du dispositif 100 ancillaire sont donc dépendantes des dimensions de prothèses à utiliser, et le même dispositif peut être produit avec une gamme de différentes tailles. Dans un mode de réalisation préféré, les lamelles donnent une forme tronconique allongée à l'ensemble de conformation 20, et comportent une forme triangulaire avant d'être planes au niveau de la zone de compression.

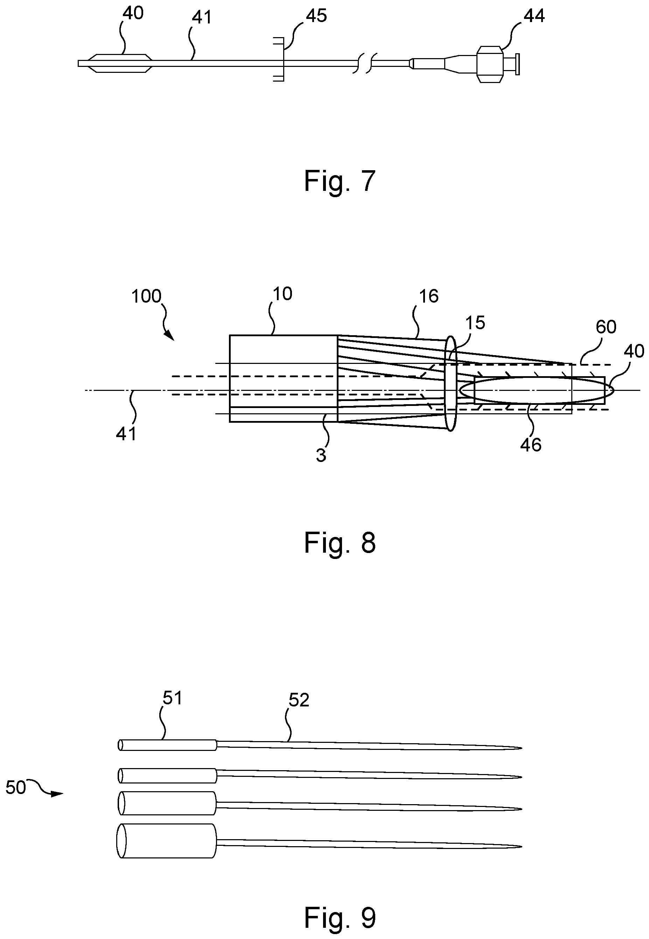

Selon le mode de réalisation illustré, le dispositif 100 ancillaire comporte une bague 15 extérieure agissant comme moyen d'actionnement de la position serrée. En effet, cette bague 15 est mobile en translation le long du dispositif, et permet de pousser l'ensemble des lamelles vers l'axe central du canal, notamment lorsque cette bague 15 est déplacée vers ou sur les lamelles 22 reliées à la partie de manipulation. À cette fin, la partie de manipulation 10 recevant les lamelles, ou une base de lamelles comporte une partie en saillie sur laquelle la bague est engagée pour comprimer radialement les lamelles. Dans le mode de réalisation illustré, cette saillie 16 prend la forme d'un diamètre extérieur de la partie de manipulation augmentant progressivement au niveau de la partie de manipulation recevant la base des lamelles. De manière alternative cette saillie 16 peut être intégrée au niveau de la base des lamelles 22 (voir figure 8). Dans les deux cas, la position serrée est activée par l'engagement de la bague 15 sur cette partie en saillie 16. Bien entendu, le matériau du dispositif ancillaire présente une certaine flexibilité permettant ce changement de position. Par exemple les lamelles sont en titane ou en acier inox 316L et la partie de manipulation comme la bague sont en résine biocompatible. Dans d'autres modes de réalisation, d'autres moyens d'actionnement configurés pour resserrer radialement les lamelles peuvent être utilisés, voir un actionnement manuel.

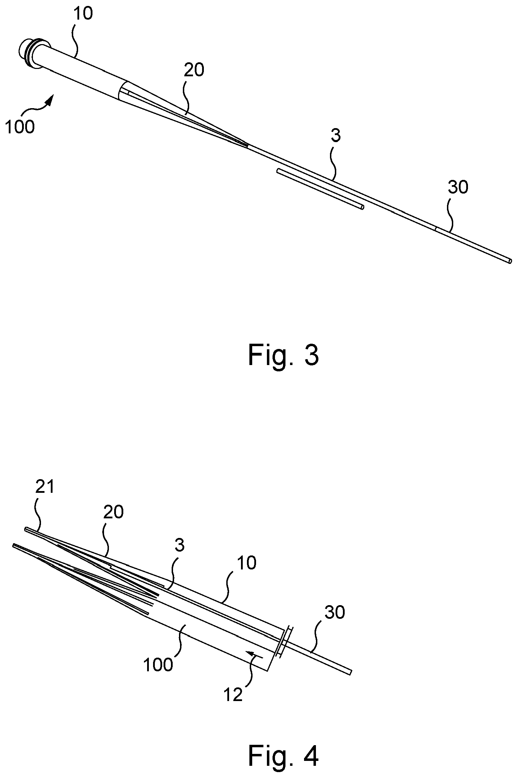

Ainsi, lors de la première anastomose, le praticien insère la prothèse le long du canal traversant, et réduit le diamètre de l'extrémité de la prothèse en activant la position serrée de l'ensemble de conformation. Le praticien utilise ensuite le dispositif ancillaire pour guider et insérer la prothèse dans l'extrémité dite haute du conduit corporel 30. Le dispositif ancillaire est alors retiré en le sortant par exemple vers l'extrémité libre de la prothèse (Fig. 3).

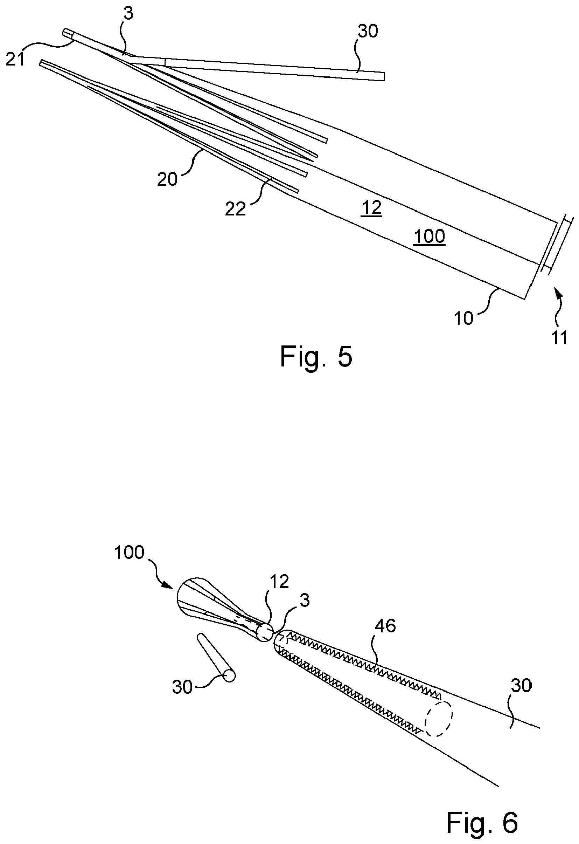

La présente invention est très avantageuse en ce qu'elle propose un dispositif ancillaire optimisé pour assister dans la deuxième anastomose également. A cette fin, l'invention propose de fournir le dispositif 100 ancillaire avec une ouverture refermable et longitudinale donnant accès au canal 12 longitudinal, et de sorte qu'un praticien puisse amener facilement l'extrémité libre de la prothèse reliée au conduit corporel jusqu'à la zone de compression 21 dudit dispositif (fig. 4). Cette ouverture permet donc de manipuler soigneusement l'extrémité libre de la prothèse pour la positionner dans ladite zone de compression et sans besoin de la faire passer par tout le corps de l'ancillaire. De plus, lorsque la longueur de la prothèse est limitée, il sera possible de positionner directement les derniers millimètres de la prothèse 3 au niveau de la zone de compression 21 et de faire sortir le reste de la prothèse 3 par l'un des espaces vides entre les lamelles 22 (fig. 5).

L'ouverture refermable est très avantageuse également en ce qu'elle facilite le retrait du dispositif ancillaire lorsque les deux anastomoses ont été effectuées, et qu'il ne reste plus d'extrémité libre vers laquelle sortir ce dispositif.

Dans un mode de réalisation préféré, l'ouverture longitudinale est configurée comme une ouverture articulée ouvrant en deux parties le corps du dispositif ancillaire (Fig. 4). Cette articulation est présente le long de la partie de manipulation et sectionne le dispositif en deux parties, avec des axes d'ouverture coïncidant avec les espaces vides entre les lamelles.

Dans un mode de réalisation, la bague 15 d'actionnement agit comme moyen de fermeture permettant de maintenir la position fermée lorsque ledit anneau est engagé au niveau de la partie de manipulation, et permettant l'ouverture lorsqu'elle est désengagée du corps du dispositif (figure 4), soit lorsqu'elle est également ouverte en deux parties. Dans un autre mode de réalisation non illustré, la partie de manipulation 20 intègre des moyens d'attache dans ladite partie de manipulation. Par exemple un crochet, un système clipsable etc.

L'invention concerne également un kit d'anastomose sans suture manuelle. Ce kit d'anastomose comportant :
- Un dispositif 100 ancillaire selon l'invention ;
- Une prothèse 3 ou substitut vasculaire ;
- Un ensemble de liaison 4 comportant au moins un manchon d'élargissement radial à picots 46 et un ballon 40 d'inflation.

Tel qu'il a été déjà mentionné, l'ensemble de liaison 4 est très avantageux en ce qu'il permet la connexion de la prothèse 3 avec le conduit corporel 30 sans besoin de suture manuelle. Comme illustré schématiquement à la figure 6, manchon d'élargissement radial à picots 46, dit aussi stent à picots, est apte à perforer la prothèse 3 et le conduit corporel 30 lors de son élargissement radial, ce qui permet de solidariser ces éléments entre eux. La présente invention, propose un ensemble de liaison 4 conçu pour coopérer avec le dispositif 100 ancillaire de l'invention, et faciliter l'intervention d'anastomose.

En particulier, l'élargissement radial du manchon d'élargissement radial à picots 46 est obtenu par inflation du ballon d'inflation à son intérieur. Pour sa part, le ballon 40 d'inflation (fig. 7) est pourvu à une extrémité d'un cathéter 41 reliant ledit ballon 40 avec une seringue d'inflation 44. Ce cathéter 41 a donc une longueur plus importante que la longueur nécessaire pour traverser le canal du dispositif 100 ancillaire. Afin d'insérer le ballon 40 et la prothèse 3 avec précision, l'invention propose de fournir l'ensemble de liaison 4 avec un système de guidage d'insertion pour définir le niveau d'insertion dudit ensemble de liaison dans le dispositif ancillaire, et dans le conduit corporel. Ce système de guidage est par exemple un jeu des éléments d'arrêt sur le corps du dispositif ancillaire et sur l'ensemble de liaison, configurés pour limiter la profondeur d'insertion de ce dernier.

Dans un mode de réalisation illustré schématiquement à la fig. 7, le cathéter 41 du ballon 40 d'inflation est pourvu d'un élément d'arrêt 45 du système de guidage sous la forme des ailettes comportant des crochets périphériques configurés pour se fixer à l'entrée 11 du dispositif ancillaire ou à une extrémité de la prothèse. L'ensemble de liaison 4 est donc inséré dans le canal du dispositif ancillaire jusqu'à son arrêt par l'ancrage des crochets des ailettes 45 du cathéter 41 au dispositif ancillaire. Les ailettes 45 ont par exemple au moins deux branches, et de préférence quatre ou six branches qui viennent s'encastrer sur la base cylindrique creuse de l'ancillaire.

Dans un autre mode de réalisation, les ailettes 45 définissent des butées configurées pour rentrer dans le canal du dispositif 100 ancillaire et être arrêtées au niveau des butées internes du dispositif ancillaire ou par une paroi de la prothèse. Dans un mode de réalisation préféré, les ailettes 45 sont mobiles en translation le long du cathéter 41. Un praticien peut ainsi limiter au cas par cas la profondeur d'insertion de l'ensemble de liaison, ce qui est très avantageux lors de la deuxième anastomose. En effet lors de la deuxième anastomose le positionnement du stent et de la prothèse doit se faire sous contrôle visuel, et au cas par cas. Lorsque ce niveau d'insertion souhaité est déterminé, le praticien pourra changer la position des ailettes de sorte qu'elles buttent contre la prothèse et l'ensemble de liaison soit immobilisé.

Dans un mode de réalisation illustré à la figure 9, le kit comprend également une enveloppe 60 de protection dudit manchon d'élargissement radial à picots 46 permettant de préserver l'intégrité des picots jusqu'à son insertion dans le conduit corporel, et notamment lors de l'étape de compression de la prothèse 3. Cette enveloppe est disposée autour du stent à picots 46, ledit stent à picots étant inséré dans la prothèse montée sur le ballon 40 et entouré par ladite enveloppe de protection. En particulier, un diamètre de l'enveloppe entourant le ballon est légèrement inférieur au diamètre de l'orifice interne au niveau de la zone de compression 21, ladite enveloppe n'est donc pas comprimée, mais uniquement l'extrémité de la prothèse devant être introduite dans le conduit corporel 30 sans pouvoir comprimer le stent à picots 46 protégé par l'enveloppe 60.

L'enveloppe 60 de protection est conservée également lors de l'étape d'insertion de la prothèse et de l'ensemble de liaison 4 dans le conduit corporel 30, pour ensuite être retirée avant l'étape d'inflation du ballon 40. Avantageusement, l'invention propose de fournir ladite enveloppe 60 avec un système pelable ou sécable permettant d'ouvrir ladite enveloppe en tirant à partir de son extrémité proximale s'étendant vers l'extérieur du conduit corporel. Sur la figure 8, les lignes pointillées indiquent des axes pelables ou sécables permettant de diviser l'enveloppe en deux parties pour faciliter son retrait. De préférence, et dans un mode de réalisation non illustré, une extrémité proximale de l'enveloppe 60 est fendue à quatre niveaux pour faciliter le pelage de l'enveloppe pour son retrait. Avantageusement, ce mode de réalisation permet également de faciliter le passage des ailettes

Enfin, le kit de l'invention comporte également un testeur 50 de diamètre comportant un jeu de corps tubulaires 51, tel que des cylindres creux 51 présentant différents diamètres, chaque cylindre 51 comportant une poignée 52 à partir de laquelle le praticien manipule le cylindre pour l'insérer dans le conduit corporel. En particulier, le praticien teste les différents cylindres jusqu'à trouver le diamètre de cylindre pouvant s'insérer dans le conduit corporel avec un jeu limité dans ledit conduit. Les diamètres des cylindres 51 sont donc légèrement inférieurs aux diamètres internes de conduits corporels pour lesquels l'anastomose est prévue. Ces cylindres peuvent permettre également de réaliser une compression externe complémentaire de la prothèse lorsque cela s'avère nécessaire.

L'invention propose ainsi un kit d'anastomose permettant de positionner parfaitement une prothèse dans un conduit corporel, ladite prothèse associée à un stent à picots pour assurer la jonction de ladite prothèse sans suture manuelle. En particulier, le dispositif de l'invention est très avantageux en ce qu'elle permet d'assister lors de la jonction de deux extrémités de la prothèse grâce à son ouverture refermable caractéristique.

## Revendications

1. Dispositif (100) ancillaire pour anastomose, ledit dispositif (100) comportant un corps allongé pourvu d'un canal (12) longitudinal et traversant destiné à recevoir à son intérieur une prothèse (3) tubulaire, le corps du dispositif comportant d'un côté proximal une partie de manipulation (10) et d'un côté distal un ensemble de conformation (20) formé par une pluralité des lamelles (22) définissant une zone de compression (21) de ladite prothèse, **caractérise en ce que** :
- le corps du dispositif comporte une ouverture longitudinale refermable donnant accès au canal (12) longitudinal destiné à recevoir ladite prothèse (3).

2. Dispositif (100) selon la revendication 1, dans lequel l'ouverture longitudinale est configurée comme une ouverture articulée ouvrant en deux parties ledit corps.

3. Dispositif selon l'une des revendications précédentes, dans lequel le dispositif comporte une configuration ouverte, et une configuration fermée définies respectivement par l'ouverture ou la fermeture de ladite ouverture longitudinale, et un moyen de verrouillage de ladite configuration fermée.

4. Dispositif selon l'une des revendications précédentes, les lamelles (22) de l'ensemble de conformation (20) sont disposées de manière circonférentielle et selon des intervalles prédéfinis autour de la partie de manipulation (10) et suivant un axe longitudinal du dispositif (100), et dans lequel dispositif les lamelles (22) comportent une position relâchée et une position serrée définissant respectivement un diamètre initial et un diamètre réduit du canal (12) recevant la prothèse (3) au niveau de ladite zone de compression (21).

5. Dispositif selon la revendication 4, ledit dispositif comportant un moyen d'actionnement configuré pour comprimer radialement lesdites lamelles (22) et activer leur position serrée.

6. Dispositif selon l'une des revendications précédentes, dans lequel la partie de manipulation comporte une ou plusieurs parties en saillie (16) au niveau d'une zone de jonction avec les lamelles (22) de l'ensemble de conformation, ladite partie en saillie (16) permettant de comprimer radialement les lamelles lorsqu'elle est poussée vers l'intérieur du dispositif.

7. Dispositif selon la revendication 6, comportant une bague (15) extérieure mobile en translation le long du corps du dispositif, ladite bague étant apte à s'engager sur la ou les parties en saillie (16) pour activer la position serrée de lamelles (22).

8. Kit pour anastomose comportant :
- un dispositif ancillaire (100) selon l'une des revendications précédentes ;
- une prothèse (3) ou substitut vasculaire
- un ensemble de liaison (4) comportant au moins un manchon d'élargissement radial à picots (46) et un ballon d'inflation (40).

9. Kit pour anastomose selon la revendication 8, ledit kit comportant un système de guidage d'insertion comportant un jeu des éléments d'arrêt sur le corps du dispositif ancillaire et sur l'ensemble de liaison, configurés pour limiter la profondeur d'insertion de l'ensemble de liaison dans le canal du dispositif ancillaire.

10. Kit pour anastomose selon l'une des revendications 8 ou 9, dans lequel le ballon d'inflation (40) comporte un cathéter (41) pour relier ledit ballon (40) à une seringue d'inflation (44), et ledit cathéter (41) comporte une ou des ailettes (45) configurées pour s'ancrer au corps du dispositif ancillaire (100) ou à la prothèse (3), et limiter une profondeur d'insertion de l'ensemble de liaison (4) dans le canal (12) du dispositif ancillaire.

11. Kit pour anastomose selon la revendication 10, dans lequel les ailettes (45) comportent des crochets périphériques configurés pour s'accrocher à une extrémité proximale de la partie de manipulation du dispositif ancillaire ou à une extrémité de la prothèse.

12. Kit pour anastomose selon l'une des revendications 10 ou 11, dans lequel les ailettes (45) sont mobiles en translation le long du cathéter (41) et comportent une configuration verrouillée et une configuration déverrouillée, limitant ou permettant respectivement une mobilité des ailettes (45).

13. Kit pour anastomose selon l'une des revendications 8 à 12, comportant une enveloppe (60) de protection pelable ou sécable entourant le manchon d'élargissement radial à picots (46).

14. Kit pour anastomose selon la revendication 13, dans lequel l'enveloppe (60) comporte au moins un axe longitudinal pelable ou sécable.

15. Kit pour anastomose selon l'une des revendications 8 à 14, comportant un testeur (50) de diamètre comportant au moins un corps tubulaire (51) de diamètre prédéfini relié à une poignée (52), et de préférence une pluralité des corps tubulaires (51) reliés respectivement à une poignée (52).
